(19) Europäisches Patentamt
European Patent Office
Office européen des brevets

(11) EP 1 214 932 A2

(12) # DEMANDE DE BREVET EUROPEEN

(43) Date de publication:
**19.06.2002 Bulletin 2002/25**

(51) Int Cl.[7]: **A61K 7/48**, A62D 3/00

(21) Numéro de dépôt: **01403191.8**

(22) Date de dépôt: **11.12.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **12.12.2000 FR 0016126**

(71) Demandeur: **IRFAQ**
**91140 Villebon sur Yvette (FR)**

(72) Inventeurs:
- **Goffinet, Pierre, Charles**
**91190 GIF-SUR-YVETTE (FR)**
- **Cottin-Riviere, Brigitte**
**86000 Poitiers (FR)**

(74) Mandataire: **Ulmann, Catherine Claire et al**
**Cabinet Beau de Loménie,**
**158 rue de l'Université**
**75340 Paris Cedex 07 (FR)**

(54) **Formulation décontaminante pour la décontamination d'agents toxiques épaissis**

(57) L'invention concerne une formulation décontaminante comprenant au moins un agent décontaminant et un solvant de formule générale (I)

$$R_1—O-[-(CH_2)_n—O-]-_mR_2 \qquad (I)$$

dans laquelle

- $R_1$ est un ($C_1$-$C_8$) alkyle, linéaire ou ramifié, un phényle ou un benzyle, non substitués ou substitués une ou plusieurs fois par un ($C_1$-$C_8$)alkyle, linéaire ou ramifié ;
- $R_2$ est l'hydrogène ou un ($C_1$-$C_4$)alkyle, linéaire ou ramifié ;
- n est un nombre entier de 2 à 6 ;
- m est un nombre entier de 1 à 4,

ladite formulation ayant un pH supérieur à 7 lorsqu'elle est mise en oeuvre en phase aqueuse à une concentration de 2 à 50% en poids, ainsi que son utilisation pour la décontamination d'agents toxiques épaissis et/ou adsorbés sur des surfaces non durcies.

EP 1 214 932 A2

## Description

L'invention concerne des formulations décontaminantes non agressives utilisables pour la décontamination d'agents toxiques organophosphorés ou organosoufrés en particulier pour la décontamination d'agents toxiques épaissis et/ou adsorbés sur des surfaces poreuses non durcies, notamment dans le domaine des agents de guerre chimique.

Il existe actuellement de nombreux esters organophosphorés dérivés des acides phosphoriques et phosphoniques, utilisés comme agents de guerre chimique tels que le Tabun, le Sarin ou le Soman.

Ces composés présentent une importante neurotoxicité du fait de leur pouvoir phosphorylant vis-à-vis des cholinestérases dont l'inhibition entraîne la mort par accumulation d'acétylcholine dans l'organisme.

Par ailleurs, il existe également des produits toxiques organosoufrés tels que les agents de guerre de la famille des vésicants, notamment l'ypérite.

Des compositions décontaminantes contenant du monoperoxyphtalate de magnésium (MPPM) et un agent tensioactif, de préférence cationique, sont décrites dans le brevet FR2 676 368. Différents procédés de décontamination, dont certains font appel aux peroxydes ou au MPPM, combinés à des agents tensioactifs cationiques, sont décrits dans la publication B. Ségués et al., Bull. Soc. Chim. 1996, 133, 925-937. La publication de S. Batthacharya, J. Org. Chem. 1997, 62, 2198-2204 rapporte une réaction de catalyse micellaire de décomposition de dérivés phosphorés par l'intermédiaire de microémulsions de MPPM en présence d'un agent tensioactif cationique. Différentes publications de C. Lion et al. (par exemple Bull. Soc. Chim., Belg., 1991, 100, 555) concernent l'efficacité décontaminante de combinaisons de différents peracides et de tensioactifs cationiques.

Toutefois, l'efficacité de telles compositions est fortement inhibée par la présence d'épaississants dans les agents toxiques.

En effet, la toxicité des agents toxiques organophosphorés ou organosoufrés est encore renforcée lorsqu'ils sont utilisés en mélange avec un polymère épaississant qui accroît leur adhérence sur la surface traitée, notamment lorsque l'agent toxique épaissi est mis en contact avec de l'eau en vue d'un rinçage.

Un exemple de polymère épaississant est un polyméthacrylate commercialisé sous la dénomination « Paraloïd K 125 » (Röhm & Haas).

La décontamination des surfaces est également rendue particulièrement difficile lorsqu'il s'agit de surfaces poreuses non durcies : l'agent toxique est dans ce cas peu accessible à l'agent décontaminant.

Les formulations décontaminantes selon l'invention permettent de résoudre le problème de la difficulté de la décontamination dû à la nature et/ou l'accessibilité de l'agent toxique par l'utilisation d'un agent décontaminant et d'un solvant qui, d'une part, solubilise à la fois ledit agent décontaminant et l'épaississant et, d'autre part, soit miscible à l'eau.

L'invention concerne donc, selon un premier aspect, une formulation décontaminante comprenant au moins un agent décontaminant et un solvant tel que défini ci-dessus.

De préférence, ledit solvant solubilise également l'agent toxique.

Par « agent décontaminant », on entend un agent capable de détruire la structure de l'agent toxique.

Avantageusement, l'invention concerne une formulation décontaminante comprenant au moins un agent décontaminant et un solvant de formule générale (I)

$$R_1 - O\text{-}[\text{-}(CH_2)_n - O\text{-}]\text{-}_m R_2 \qquad (I)$$

dans laquelle

- $R_1$ est un ($C_1$-$C_8$) alkyle, linéaire ou ramifié, un phényle ou un benzyle, non substitués ou substitués une ou plusieurs fois par un ($C_1$-$C_8$)alkyle, linéaire ou ramifié ;
- $R_2$ est l'hydrogène ou un ($C_1$-$C_4$)alkyle, linéaire ou ramifié ;
- n est un nombre entier de 2 à 6 ;
- m est un nombre entier de 1 à 4.

De préférence, ladite formulation décontaminante a un pH supérieur à 7, de préférence de 8,5 à 12, lorsqu'elle est mise en oeuvre en phase aqueuse à une concentration de 2 à 50% en poids.

Avantageusement, ledit solvant est peu ou pas inflammable, et présente de préférence un point éclair en coupe fermée supérieur ou égal à 50°C.

Le point éclair correspond à la température à laquelle un produit qui se trouve en enceinte fermée et à pression atmosphérique s'enflamme lorsqu'on en approche une flamme, après augmentation progressive de la température. Les mesures sont effectuées à l'aide d'un appareil Sétaflash ®.

Des solvants préférés sont par exemple :

- l'éther monopropylique du propylèneglycol (Dowanol PnP),
- l'éther monopropylique du dipropylèneglycol (Dowanol DPnP),
- l'éther diméthylique du dipropylèneglycol (Proglyde DMM),
- l'éther monobenzylique du tétraéthylèneglycol (phénol éthoxylé à environ 4 oxydes d'éthylène tel que l'Ethylan HB4 ® commercialisé par Akzo Nobel), et
- l'éther monobutylique du diéthylèneglycol (butyldiglycol).

Selon un aspect avantageux, le ou les agent(s) décontaminant(s) est (sont) compris dans une composition décontaminante.
Selon un aspect préféré, les formulations décontaminantes selon l'invention comprennent ainsi :

- une composition décontaminante constituée d'une composition sous forme de poudre comprenant au moins un agent décontaminant, ladite composition étant le cas échéant mise en solution, en dispersion, en émulsion, en microémulsion ou en suspension dans l'eau à raison de 2 à 50% en poids de poudre dans l'eau, et
- un solvant tel que défini plus haut.

Les compositions décontaminantes utilisables selon l'invention sont de préférence mises en oeuvre en phase aqueuse sous forme de solution, de dispersion, d'émulsion, de microémulsion ou de suspension dans l'eau à une concentration de 2 à 50 %, de préférence de 8 à 25 % en poids, ladite phase aqueuse ayant un pH supérieur à 7, de préférence compris entre 8,5 et 12.
Le solvant tel que défini plus haut est dans ce cas présent dans la formulation décontaminante selon l'invention à raison de 3 à 60 % en poids de la composition décontaminante en phase aqueuse.
Par « formulation décontaminante », on entend aussi bien une formulation comprenant le mélange de la composition décontaminante et du solvant dans un même conditionnement, que l'association de la composition décontaminante en phase aqueuse et du solvant dans des conditionnements séparés.
La composition décontaminante comprend au moins un agent décontaminant et, de manière optionnelle, au moins un agent tensioactif cationique et/ou au moins un agent tensioactif non ionique. D'autres composants optionnels peuvent être choisis parmi un tampon alcalin, un agent hydrotrope, un agent séquestrant stabilisant et un agent antimousse.
Les différents composants de la composition décontaminante sont détaillés ci-dessous :
L'agent décontaminant selon l'invention peut être un agent peroxydant organique ou minéral.
Par « un agent peroxydant », on entend dans la suite de la description aussi bien un seul agent peroxydant qu'un mélange d'agents peroxydants.
Dans un aspect avantageux, l'agent peroxydant est un agent peroxydant organique tel qu'un peracide ou un peroxyde, par exemple le monoperoxyphtalate de magnésium hexahydraté (MPPM) ; l'acide diperoxydodécanedioïque (DPDA) ; l'acide perbenzoïque, l'acide perphtalique, l'acide perlaurique, l'acide perazélaïque, l'acide persalicylique, l'acide diperadipique, l'acide peracétique, l'acide performique, l'acide diperoxytéréphtalique et leurs sels ; le peroxyde de dicumyle, le peroxyde de tert-butylcumyle et le peroxyde de diphtaloyle. Le MPPM est un agent décontaminant particulièrement avantageux.
On peut également utiliser le peroxyde d'hydrogène ($H_2O_2$) ou un agent peroxydant minéral tel que :

- les adduits de $H_2O_2$ tels que les percarbonates, en particulier le percarbonate de sodium ; le 1,4-diazabicyclo [2.2.2]octane$(H_2O_2)_2$ ; les peroxyhydrates de phosphates tel que le peroxypyrophosphate de sodium ; la perurée ;
- les persels tels que le perborate de sodium tétrahydraté ou monohydraté ; le monopersulfate de potassium, le Curox ® et les sels d'acide de Caro, le persulfate d'ammonium, le persulfate de sodium ou le persulfate de potassium ; le permanganate de potassium ; les peroxymonophosphates ; les peroxydiphosphates ;
- les peroxydes alcalins ou alcalino-terreux tels que le peroxyde de sodium ou de magnésium.

Dans un aspect avantageux, on mettra en oeuvre un agent peroxydant minéral en présence d'un activateur d'oxydation, c'est-à-dire une molécule capable de générer *in situ* un peracide.
Dans ce cas, on peut utiliser notamment un activateur sans propriétés interfaciales tels que ceux qui génèrent :

- l'acide peracétique, par exemple la tétraacétyléthylènediamine (TAED), le pentaacétylglucose (PAG), le tétraacétylglucoluryl (TAGU), l'acide tétraacétylcyanurique (TACA), l'α-acétoxy-α-méthyl N-N'(diacétyl)malonamide, l'acide acétylsalicylique (ASA), le paraacétoxybenzènesulfonate de sodium (AOBS), la diacétyldiméthylglyoxine (DDG) et l'éthylidène benzoateacétate (EBA) ;
- l'acide perphtalique tel que l'anhydride phtalique (PAN) ; ou
- l'acide perbenzoïque tel que le benzoylimidazole (BID), le parabenzoxybenzènesulfonate de sodium (BOBS).

On peut également utiliser des activateurs avec propriétés interfaciales tels que le nonanoyloxybenzènesulfonate (NOBS), qui génère de l'acide pernonanoïque ou de l'isononanoyloxybenzènesulfonate (ISONOBS), qui génère de l'acide perisononanoïque.

Une association préférée est constituée par le percarbonate de sodium ou la perurée en tant qu'agents peroxydants minéraux et la TAED en tant qu'activateur de peroxydation.

L'agent décontaminant peut également être choisi parmi les agents de blanchiment halogénés ou les précurseurs d'agents de blanchiment halogénés, tels que l'hypochlorite de sodium, de potassium ou de calcium, le dichloroisocyanurate de sodium ou de calcium, le trichloroisocyanurate de sodium, etc.

Par « agent tensioactif cationique » que peut comprendre la composition décontaminante définie plus haut, on entend dans la suite de la description aussi bien un seul type d'agent tensioactif cationique qu'un mélange d'agents tensioactifs cationiques.

En tant qu'agent tensioactif cationique, on peut utiliser par exemple le chlorure ou le bromure de cocoyltriméthylammonium, le chlorure de didécyldiméthylammonium, le chlorure de benzalkonium, les mélanges de chlorures de benzalkonium et de chlorures d'alkyldiméthyléthylbenzylammonium, et les agents tensioactifs dérivés d'ammonium quaternaires ou polyquaternaires, éventuellement partiellement éthoxylés ou dont la chaîne alkyle est éventuellement interrompue par un groupement ester ou amide, ainsi que leurs mélanges.

D'une manière générale, on peut utiliser des agents tensioactifs cationiques représentés par les formules

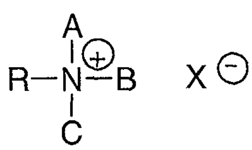 ou 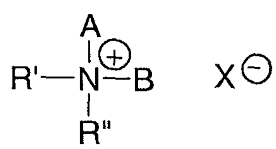

dans lesquelles

- R est un alkyle ou un alcényle en $C_8$-$C_{22}$
- R' et R", indépendamment, représentent un alkyle ou un alcényle en $C_8$-$C_{22}$
- C est

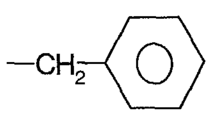 ; 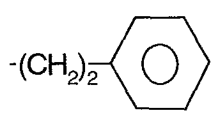 ; $CH_3$ ou 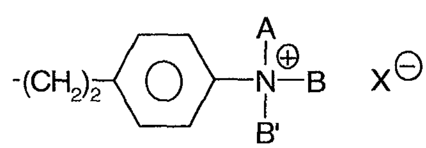 ;

- A, B et B' représentent indépendamment $CH_3$, $CH_2CH_3$, $C(CH_3)_3$ ou

$$\left[ (CH_2)_mO \right]_n - H ,$$

  m étant 2 ou 3 et n étant un nombre entier de 1 à 3,
- $X^{\ominus}$ représente un halogène ou un groupe méthosulfate.

  On peut utiliser également des agents tensioactifs polycationiques tels que le dichlorure de N,N,N',N',N'-pentaméthyl-N-suif-1,3-propane diammonium et le trichlorure de N,N,N',N',N',N",N"-heptaméthyl-N-suif-dipropylène triammonium.

  Par « un agent tensioactif non ionique », on entend dans la suite de la description aussi bien un seul type d'agent tensioactif non ionique qu'un mélange d'agents tensioactifs non ioniques.

  En tant qu'agent tensioactif non ionique utilisable de manière optionnelle, notamment à raison de 0 à 25%, de préférence 5 à 20%, on peut citer par exemple

- les alcools gras alcoxylés de formule

$$R-[(CH_2)_2O]_n-[(CH_2)_3O]_m-H$$

dans laquelle R est tel que défini ci-dessus ; n et m, indépendamment, représentent un nombre entier de 0 à 50, dont la somme n+m ≥ 1;

- les alkylpolyglucosides de formule R'''-[glucose]$_n$- dans laquelle R''' est un alkyle en $C_8$-$C_{16}$ et n est un nombre entier de 1 à 3;
- les esters de sucres réduits, les esters de polyols et leurs dérivés éthoxylés, tels que par exemple le monostéarate de sorbitan, de glycérol ou d'éthylèneglycol, ou le monostéarate de sorbitan éthoxylé à 20 oxydes d'éthylène ;
- les alkylamides éthoxylés de formule :

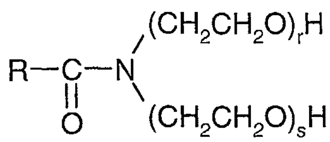

  où R est tel que défini ci-dessus et r et s sont des nombres entiers de 0 à 15, dont la somme r + s > 1 ;
- les alkylpyrrolidones, dont le groupement alkyle est en $C_6$-$C_{20}$ ;
- et leurs mélanges.

Des agents tensioactifs non ioniques préférés sont l'oxyde de lauryldiméthylamine et l'octylpyrrolidone.
La composition décontaminante comprise dans la formulation décontaminante selon l'invention peut comprendre un tampon alcalin.
Par « un tampon alcalin », on entend dans la suite de la description aussi bien un seul tampon alcalin qu'un mélange de tampons alcalins.
Un tampon alcalin avantageux dans les compositions utilisées selon l'invention est par exemple un carbonate ou un silicate alcalin, notamment le carbonate de sodium. Avantageusement, les solutions contenant les compositions selon l'invention ont un pH supérieur à 7, de préférence compris entre 8,5 et 12, notamment entre 9 et 11. Si nécessaire, le pH peut être ajusté à l'aide d'un agent alcalinisant tel que l'hydroxyde de sodium, de potassium, d'ammonium ; les silicates ou les borates de sodium, de potassium, d'ammonium etc ...
La composition décontaminante comprise dans la formulation décontaminante selon l'invention peut comprendre un agent hydrotrope.
Par « un agent hydrotrope », on entend dans la suite de la description aussi bien un seul type d'agent hydrotrope qu'un mélange d'agents hydrotropes.
En tant qu'agent hydrotrope, on peut utiliser par exemple l'urée, les cumènesulfonates, toluènesulfonates ou xylènesulfonates de sodium, potassium ou ammonium.
La composition décontaminante comprise dans la formulation décontaminante selon l'invention peut comprendre un agent séquestrant stabilisant.
Par « un agent séquestrant stabilisant » que peut comprendre la composition décontaminante définie plus haut, on entend dans la suite de la description aussi bien un seul type d'agent séquestrant stabilisant qu'un mélange d'agents séquestrants stabilisants.
L'agent séquestrant stabilisant est avantageusement choisi parmi le gluconate de sodium, le citrate de sodium, le diéthylènetriaminepentaméthylènephosphonate de sodium (DETPM), l'éthylènediaminetétraméthylènephosphonate de sodium (EDTMP), l'hydroxyéthyldiéthylphosphonate de sodium (HEDP), les aminopolycarboxylates tels que le nitrilotriacétate de sodium (NTA), la diéthylènetriaminepentaacétate de sodium (DTPA), etc.
En tant qu'agent antimousse pouvant être utilisé dans la composition décontaminante comprise dans la formulation décontaminante selon l'invention, on peut citer par exemple un polydiméthylsiloxane.
Dans la suite de la description, les pourcentages sont exprimés en pourcentages pondéraux.
Les compositions décontaminantes en phase aqueuse telles que définies plus haut peuvent également contenir des agents antigels, à raison de 5 à 40 %, de préférence de 15 à 25 %, tels que par exemple le propylèneglycol, l'éthylèneglycol ou le diéthylèneglycol.
Selon un aspect préféré, l'invention concerne une formulation décontaminante comprenant :

- une composition décontaminante en phase aqueuse comprenant, en pourcentages pondéraux,

  - 1 à 98,5 %, de préférence 10 à 70 %, d'un agent peroxydant,
  - 0 à 25 %, de préférence 5 à 15 %, d'un agent activateur de peroxydation,
  - 0,5 à 90 %, de préférence 2 à 30 %, d'un tampon alcalin,

- 0 à 30 %, de préférence 1 à 20 %, d'un agent tensioactif cationique et/ou non ionique,
- 0 à 50 %, de préférence 2 à 30 %, d'un agent hydrotrope,
- 0 à 35 %, de préférence 15 à 20 %, d'un agent séquestrant stabilisant,
- 0 à 5 %, de préférence 0,005 à 1 %, d'antimousse,

la somme des constituants étant égale à 100 %, à raison de 2 à 50 % de poudre dans de l'eau,

• un solvant de formule (I)

$$R_1—O\text{-}[\text{-}(CH_2)_n—O\text{-}]\text{-}_mR_2 \qquad (I)$$

dans laquelle

- $R_1$ est un ($C_1$-$C_8$) alkyle, linéaire ou ramifié, un phényle ou un benzyle, non substitués ou substitués une ou plusieurs fois par un ($C_1$-$C_8$)alkyle, linéaire ou ramifié ;
- $R_2$ est l'hydrogène ou un ($C_1$-$C_4$)alkyle, linéaire ou ramifié ;
- n est un nombre entier de 2 à 6 ;
- m est un nombre entier de 1 à 4,

à raison de 3 à 60 % de la composition décontaminante en phase aqueuse.

Avantageusement, ladite composition décontaminante comprend, en pourcentages pondéraux :

- 10 à 78 % d'un agent peroxydant,
- 0 à 15 % d'un agent activateur de peroxydation,
- 2 à 30 % d'un tampon alcalin,
- 5 à 20 % d'un agent tensioactif cationique,
- 0 à 20 % d'un agent tensioactif non ionique,
- 0 à 30 % d'un agent hydrotrope,
- 15 à 20 % d'un agent séquestrant stabilisant, et
- 0 à 1 %, d'antimousse,

la somme des constituants étant égale à 100 %.

Une composition décontaminante préférée aux fins de l'invention comprend, en pourcentages pondéraux :

- 30 à 70 % de MPPM,
- 10 à 30 % de gluconate de sodium ou de citrate de sodium,
- 10 à 30 % de carbonate de sodium,
- 0 à 5 % d'hydroxyde de sodium,
- 5 à 10 % de chlorure de benzalkonium,
- 0 à 1 % d'antimousse,

la somme des constituants étant égale à 100 %.

Une autre composition décontaminante avantageuse aux fins de l'invention comprend, en pourcentages pondéraux :

- 10 à 40 % de percarbonate de sodium,
- 5 à 15 % de TAED,
- 2 à 20 % de carbonate de sodium,
- 10 à 30 % de mélange de chlorures de benzalkonium,
- 0 à 30 % d'urée,
- 2 à 20 % de DETPM ou de gluconate de sodium, et
- 0 à 1 % d'antimousse,

la somme des constituants étant égale à 100 %.

Des formulations décontaminantes particulièrement préférées comprennent une composition décontaminante telle que définie ci-dessus et un solvant choisi parmi :

- l'éther monopropylique du propylèneglycol (Dowanol PnP),
- l'éther monopropylique du dipropylèneglycol (Dowanol DPnP),
- l'éther diméthylique du dipropylèneglycol (Proglyde DMM),
- l'éther monobenzylique du tétraéthylèneglycol (phénol éthoxylé à environ 4 oxydes d'éthyle tel que l'Ethylan HB4 ® commercialisé par Akzo Nobel), et
- l'éther monobutylique du diéthylèneglycol (butyldiglycol).

Les composants de la formulation finale se présentant sous forme liquide à température ambiante peuvent être, soit pulvérisés sur la poudre constituant le reste de la formulation, soit incorporés séparément sous forme liquide dans la solution décontaminante.

Les formulations décontaminantes peuvent être utilisées de manière manuelle ou mécanique par aspersion, badigeon, pulvérisation, trempage, imprégnation ou toute autre opération permettant le contact desdites compositions avec du matériel ou des personnes contaminés.

Selon un aspect avantageux, l'invention concerne également l'utilisation des formulations décontaminantes telles que définies ci-dessus pour la décontamination d'agents toxiques épaissis et/ou adsorbés sur des surfaces poreuses non durcies.

La stabilité des solutions est mesurée par dosage de l'oxygène actif après une durée de stockage donnée (1h30) à température ambiante ou à 40°C, selon la nature de l'agent oxydant.

Les mesures d'efficacité décontaminante sont effectuées par analyse du toxique résiduel par chromatographie en phase gazeuse.

L'invention est illustrée par les exemples ci-après :

<u>Exemple 1 :</u>

Différentes formulations selon l'invention sont rapportées dans le tableau 1 ci-dessous. Les quantités sont exprimées en pourcentages pondéraux.

Tableau 1

| COMPOSITION DECONTAMINANTE | A0 | A1 | A2 | A3 | A4 | A5 | A6 | A7 | A8 |
|---|---|---|---|---|---|---|---|---|---|
| Monoperoxyphtalate de magnésium | | | | | | | | | |
| Percarbonate de sodium | 4,40 | 4,40 | 4,40 | 4,40 | 4,40 | 4,40 | 4,40 | 4,40 | 4,40 |
| Tétraacétyl-éthylènediamine | 2,40 | 2,40 | 2,40 | 2,40 | 2,40 | 2,40 | 2,40 | 2,40 | 2,40 |
| Chlorure de benzalkonium | 1,00 | 1,00 | 2,00 | 1,00 | 1,00 | 2,00 | 1,00 | 1,00 | 1,00 |
| Carbonate de sodium | 1,00 | 1,00 | 1,00 | 1,00 | 1,00 | 1,00 | 1,00 | 1,00 | 1,00 |
| Urée | 4,30 | 4,30 | 4,30 | 4,30 | 4,30 | 4,30 | 4,30 | 4,30 | 4,30 |
| Hydroxyde de sodium | | | | | | | | | |
| Ethylènediamine-tétraméthylènephosphonate de sodium | | | | 4,00 | | | | | |
| Diéthylènetriamine-pentaméthylènephosphonate de sodium | | | 4,00 | | | | | | |
| Citrate de sodium | | | | | | 4,00 | | | |
| Gluconate de sodium | | | | | 4,00 | | | | |
| Ethylènediamine-tétraacétate de sodium | | | | | | | 4,00 | 4,00 | |
| Diéthylènetriamine-pentaacétate de sodium | | | | | | | | | 4,00 |
| Sulfate de magnésium | | | | | | | | 1,00 | |
| Huile de silicone DB 100 (Dow Corning) | 0,01 | 0,01 | 0,01 | 0,01 | 0,01 | 0,01 | 0,01 | 0,01 | 0,01 |
| | | | | | | | | | |
| Total poudre | 13,11 | 13,11 | 18,11 | 17,11 | 17,11 | 18,11 | 17,11 | 18,11 | 17,11 |
| | | | | | | | | | |
| Eau à 28°HF (temp. = 40°C) à ajuster jusqu'à 100 | 86,89 | 86,89 | 81,89 | 82,89 | 82,89 | 81,89 | 82,89 | 81,89 | 82,89 |
| | | | | | | | | | |
| **Total** | **100,00** | **100,00** | **100,00** | **100,00** | **100,00** | **100,00** | **100,00** | **100,00** | **100,00** |
| **SOLVANT** | | | | | | | | | |
| Dowanol PnP | 11 | | | | | | | | |
| Dowanol DPnP | | 8 | | | | 5 | | | |
| Proglyde DMM | | | 15 | | | | | | |
| Phénol à 4 oxydes d'éthylène | | | | 15 | | | | | |
| Butyl diglycol | | | | | 15 | | 40 | 5 | |
| Butyltriéthylène glycol | | | | | | | | | 10 |

Tableau 1 (suite)

| COMPOSITION DECONTAMINANTE | B0 | B1 | B2 | B3 | B4 | B5 | B6 | B7 | B8 |
|---|---|---|---|---|---|---|---|---|---|
| Monoperoxyphtalate de magnésium | 8,00 | 8,00 | 8,00 | 8,00 | 8,00 | 8,00 | 8,00 | 8,00 | 8,00 |
| Percarbonate de sodium | | | | | | | | | |
| Tétraacétyl-éthylènediamine | | | | 4,00 | | | | | |
| Chlorure de benzalkonium | 1,30 | 1,30 | 1,30 | 1,30 | 1,30 | 1,30 | 1,30 | 1,30 | 1,30 |
| Carbonate de sodium | 3,00 | 3,00 | 3,00 | 3,00 | 3,00 | 3,00 | 3,00 | 3,00 | 3,00 |
| Urée | | | | | | | | | |
| Hydroxyde de sodium | 0,60 | 0,60 | 0,60 | 0,60 | 0,60 | 0,60 | 0,60 | 0,60 | 0,60 |
| Ethylènediamine-tétraméthylènephosphonate de sodium | | | | 4,00 | | | | | |
| Diéthylènetriamine-pentaméthylènephosphonate de sodium | | | 4,00 | | | | | | |
| Citrate de sodium | | | | | | 4,00 | | | |
| Gluconate de sodium | | | | | 4,00 | | | | |
| Ethylènediamine-tétraacétate de sodium | | | | | | | 4,00 | 4,00 | |
| Diéthylènetriamine-pentaacétate de sodium | | | | | | | | | 4,00 |
| Sulfate de magnésium | | | | | | | | 1,00 | |
| Huile de silicone DB 100 (Dow Corning) | 0,01 | 0,01 | 0,01 | 0,01 | 0,01 | 0,01 | 0,01 | 0,01 | 0,01 |
| | | | | | | | | | |
| Total poudre | 12,91 | 12,91 | 16,91 | 16,91 | 16,91 | 16,91 | 16,91 | 17,91 | 16,91 |
| | | | | | | | | | |
| Eau à 28°HF (temp. = 20°C) | 87,09 | 87,09 | 83,09 | 83,09 | 83,09 | 83,09 | 83,09 | 82,09 | 83,09 |
| | | | | | | | | | |
| **Total** | **100,00** | **100,00** | **100,00** | **100,00** | **100,00** | **100,00** | **100,00** | **100,00** | **100,00** |
| **SOLVANT** | | | | | | | | | |
| Dowanol PnP | 11 | | | | | | | | |
| Dowanol DPnP | | 8 | | | | 5 | | | |
| Proglyde DMM | | | 15 | | | | | | |
| Phénol à 4 oxydes d'éthylène | | | | 15 | | | | | |
| Butyl diglycol | | | | | 15 | | 40 | 5 | |
| Butyltriéthylène glycol | | | | | | | | | 10 |
| | | | | | | | | | |

Exemple 2 :

L'efficacité des solutions décontaminantes contenant des compositions du tableau 1 ci-dessus a été mesurée par analyse du toxique résiduel par chromatographie en phase gazeuse (CPG).

La mesure de l'efficacité décontaminante a été effectuée sur la demi-ypérite (2-hydroxyéthyl 2-phényléthyl sulfure) épaissie avec 3 % de Paraloïd K 125. Les essais sont effectués à une concentration de 200 mg de substance toxique /55 ml de formulation décontaminante 90 min après la mise en solution et avec un temps de contact de la formulation décontaminante et de la substance toxique de 30 min.

Le toxique épaissi est déposé sur des plaques métalliques recouvertes de peintures glycérophtaliques. La mesure de l'efficacité décontaminante est donnée par l'extraction du toxique, d'une part sur la plaque (décontamination plaque) et d'autre part dans la solution (décontamination globale), par un solvant organique. Les phases organiques sont percolées avant d'être injectées en CPG, afin de bloquer l'épaississant.

Parallèlement à cette mesure d'efficacité, on mesure l'oxygène actif après 15 min et 90 min (mesure de stabilité).

Les essais ont été réalisés selon la méthode suivante :

1/ Mesure de l'efficacité décontaminante

La formulation décontaminante est préparée de façon classique ; les poudres sont solubilisées dans le mélange eau + solvant à température ambiante (pour les décontaminants à base de MPPM) ou à 40°C (pour les poudres à base de PC-TAED). La solution décontaminante est ensuite laissée à reposer 90 min.

60 min après la préparation de la formulation décontaminante, le toxique épaissi est déposé à l'aide d'une pipette automatique sur une plaque métallique recouverte de peinture glycérophtalique. Il est important à ce niveau de connaître la masse exacte de toxique déposé.

Le décontaminant est alors transféré dans la boîte de décontamination. La plaque (sur laquelle a été déposé le toxique) est ensuite placée dans la solution pour 30 min.

Lorsque les 30 min sont écoulées, on verse dans la boîte 3 ml d'une solution saturée en thiosulfate de sodium pour arrêter la décontamination. On verse ensuite la phase aqueuse dans une ampoule à décanter et on réalise les extractions classiques (deux extractions successives par 5 ml de mélange acétate d'éthyle - cyclohexane). Le volume de phase organique récoltée est mesuré. On replace la plaque dans la boîte et on verse 20 ml de mélange acétate d'éthylecyclohexane sur les parois de la plaque et de la boîte afin de solubiliser la demi-ypérite épaissie. On laisse ensuite tremper environ 30 min, puis on récupère la phase organique (il reste probablement un peu de phase aqueuse au fond de la boîte).

Lorsque toutes les extractions ont été effectuées, on prépare une mini-colonne par échantillon (un peu de coton, 15 mm de silice et 2 mm de sable de Fontainebleau dans une pipette Pasteur, humidification par l'éluant habituel), puis on prélève 1 ml de chaque échantillon et on le verse dans une mini-colonne afin de les percoler. On ajoute une quantité connue de dodécanol dans chaque échantillon percolé, puis on les injecte en CPG.

Le pourcentage de décomposition du toxique est donné par la formule

$$\left(1 - \frac{\text{masse de toxique résiduel (mg)}}{\text{masse de toxique de départ (mg)}}\right) \times 100$$

Les résultats sont rapportés dans le tableau 2 ci-après :

Tableau 2

| SOLVANT DE LA FORMULATION | % décontamination globale | % décontamination plaque |
|---|---|---|
| Sans | 28 % | 12 % |
| 11 % Dowanol PnP | 64 % | 84 % |
| 8 % Dowanol DPnP | 74 % | 92 % |
| 15 % Proglyde DMM | 54 % | 85 % |
| 15 % Phénol à 4 oxydes d'éthylène | non mesuré | 88 % |
| 15 % Butyldiglycol | 96 % | 93 % |

Les résultats montrent que les formulations décontaminantes selon l'invention présentent une efficacité décontaminante significativement supérieure à celle d'une formulation ne contenant pas de solvant.

**Revendications**

1. Formulation décontaminante comprenant au moins un agent décontaminant et un solvant de formule générale (I)

$$R_1—O-[-(CH_2)_n—O-]-_mR_2 \quad (I)$$

dans laquelle

- $R_1$ est un ($C_1$-$C_8$) alkyle, linéaire ou ramifié, un phényle ou un benzyle, non substitués ou substitués une ou plusieurs fois par un ($C_1$-$C_8$)alkyle, linéaire ou ramifié ;
- $R_2$ est l'hydrogène ou un ($C_1$-$C_4$)alkyle, linéaire ou ramifié ;
- n est un nombre entier de 2 à 6 ;
- m est un nombre entier de 1 à 4,

ladite formulation ayant un pH supérieur à 7 lorsqu'elle est mise en oeuvre en phase aqueuse à une concentration de 2 à 50% en poids.

2. Formulation selon la revendication 1, **caractérisée en ce que** ledit solvant est peu ou pas inflammable, et présente de préférence un point éclair en coupe fermée supérieur ou égal à 50°C.

3. Formulation selon les revendications 1 ou 2, **caractérisée en ce que** ledit solvant est choisi parmi :

- l'éther monopropylique du propylèneglycol,
- l'éther monopropylique du dipropylèneglycol,
- l'éther diméthylique du dipropylèneglycol,
- l'éther monobenzylique du tétraéthylèneglycol, et
- l'éther monobutylique du diéthylèneglycol (butyldiglycol).

4. Formulation selon l'une quelconque des revendications 1 à 3, **caractérisée en ce que** le ou les agent(s) décontaminant(s) est (sont) compris dans une composition décontaminante.

5. Formulation selon l'une quelconque des revendications 1 à 4, **caractérisée en ce qu'**elle comprend :

- une composition décontaminante constituée d'une composition sous forme de poudre comprenant au moins un agent décontaminant, ladite composition étant le cas échéant mise en solution, en dispersion, en émulsion, en microémulsion ou en suspension dans l'eau à raison de 2 à 50% en poids de poudre dans l'eau, et
- un solvant tel que défini selon les revendications 1 à 4.

6. Formulation selon l'une quelconque des revendications 1 à 5, **caractérisée en ce qu'**elle comprend une composition décontaminante mise en oeuvre en phase aqueuse sous forme de solution, dispersion, émulsion, microémulsion ou suspension dans l'eau à une concentration de 2 à 50 %, de préférence de 8 à 25 % en poids, ladite phase aqueuse ayant un pH supérieur à 7, de préférence compris entre 8,5 et 12 et un solvant à raison de 3 à 60 % en poids de la composition décontaminante en phase aqueuse.

7. Formulation selon l'une quelconque des revendications 1 à 6, **caractérisée en ce que** la composition décontaminante comprend au moins un agent décontaminant et, de manière optionnelle, au moins un agent tensioactif cationique et/ou au moins un agent tensioactif non ionique, un tampon alcalin, un agent hydrotrope, un agent séquestrant stabilisant et un agent antimousse.

8. Formulation selon l'une quelconque des revendications 1 à 7, **caractérisée en ce qu'**elle comprend, en pourcentages pondéraux :

• une composition décontaminante en phase aqueuse comprenant

- 1 à 98,5 %, de préférence 10 à 70 %, d'un agent peroxydant,
- 0 à 25 %, de préférence 5 à 15 %, d'un agent activateur de peroxydation,
- 0,5 à 90 %, de préférence 2 à 30 %, d'un tampon alcalin,
- 0 à 30 %, de préférence 1 à 20 %, d'un agent tensioactif cationique et/ou non ionique,
- 0 à 50 %, de préférence 2 à 30 %, d'un agent hydrotrope,
- 0 à 35 %, de préférence 15 à 20 %, d'un agent séquestrant stabilisant,
- 0 à 5 %, de préférence 0,005 à 1 %, d'antimousse,

la somme des constituants étant égale à 100 %, à raison de 2 à 50 % de poudre dans de l'eau,

- un solvant de formule (I)

$$R_1—O\text{-}[\text{-}(CH_2)_n—O\text{-}]\text{-}_mR_2 \qquad (I)$$

dans laquelle

- $R_1$ est un ($C_1$-$C_8$) alkyle, linéaire ou ramifié, un phényle ou un benzyle, non substitués ou substitués une ou plusieurs fois par un ($C_1$-$C_8$)alkyle, linéaire ou ramifié ;
- $R_2$ est l'hydrogène ou un ($C_1$-$C_4$)alkyle, linéaire ou ramifié ;
- n est un nombre entier de 2 à 6 ;
- m est un nombre entier de 1 à 4,

à raison de 3 à 60 % de la composition décontaminante en phase aqueuse.

**9.** Formulation selon la revendication 8, **caractérisée en ce que** ladite composition décontaminante comprend, en pourcentages pondéraux :

- 10 à 78 % d'un agent peroxydant,
- 0 à 15 % d'un agent activateur de peroxydation,
- 2 à 30 % d'un tampon alcalin,
- 5 à 20 % d'un agent tensioactif cationique,
- 0 à 20 % d'un agent tensioactif non ionique,
- 0 à 30 % d'un agent hydrotrope,
- 15 à 20 % d'un agent séquestrant stabilisant, et
- 0 à 1 %, d'antimousse,

la somme des constituants étant égale à 100 %.

**10.** Formulation selon les revendications 8 ou 9, **caractérisée en ce que** ladite composition décontaminante comprend, en pourcentages pondéraux :

- 30 à 70 % de MPPM,
- 10 à 30 % de gluconate de sodium ou de citrate de sodium,
- 10 à 30 % de carbonate de sodium,
- 0 à 5 % d'hydroxyde de sodium,
- 5 à 10 % de chlorure de benzalkonium,
- 0 à 1 % d'antimousse,

la somme des constituants étant égale à 100 %.

**11.** Formulation selon les revendications 8 ou 9, **caractérisée en ce que** ladite composition décontaminante comprend, en pourcentages pondéraux :

- 10 à 40 % de percarbonate de sodium,
- 5 à 15 % de TAED,
- 2 à 20 % de carbonate de sodium,
- 10 à 30 % de mélange de chlorures de benzalkonium,

- 0 à 30 % d'urée,
- 2 à 20 % de DETPM ou de gluconate de sodium, et
- 0 à 1 % d'antimousse,

la somme des constituants étant égale à 100 %.

**12.** Formulation selon l'une quelconque des revendications 1 à 11, **caractérisée en ce qu'**elle comprend le mélange de la composition décontaminante et du solvant dans un même conditionnement ou l'association de la composition décontaminante en phase aqueuse et du solvant dans des conditionnements séparés.

**13.** Utilisation d'une formulation selon l'une quelconque des revendications 1 à 12 pour la décontamination d'agents toxiques épaissis et/ou adsorbés sur des surfaces non durcies.